Europäisches Patentamt

(19) **European Patent Office**

**Office européen des brevets**

(11) Publication number: **0 032 304**
**B1**

(12) **EUROPEAN PATENT SPECIFICATION**

(45) Date of publication of patent specification: **15.08.84**

(21) Application number: **80304644.0**

(22) Date of filing: **19.12.80**

(51) Int. Cl.³: **A 61 K  31/73,**
**A 61 K  31/71, A 23 K  1/17**
**//(A61K31/73, 31/71)**

(54) Antibiotic compositions.

(30) Priority: **26.12.79 US 107240**
**26.12.79 US 107239**

(43) Date of publication of application:
**22.07.81 Bulletin 81/29**

(45) Publication of the grant of the patent:
**15.08.84 Bulletin 84/33**

(84) Designated Contracting States:
**DE GB LU NL SE**

(56) References cited:
**DE - A - 1 926 804**
**GB - A - 749 485**
**GB - A - 1 402 947**
**US - A - 3 491 187**
**US - A - 3 876 767**

**UNLISTED DRUGS, vol. 29, no. 6, June 1977,**
**page 102 Chatham, N.J. USA**
**DERWENT JAPANESE PATENT REPORT, vol. S,**
**no. 45, December 21, 1971, London, GB**

(73) Proprietor: **ELI LILLY AND COMPANY**
**307, East McCarty Street**
**Indianapolis Indiana 46285 (US)**

(72) Inventor: **Ose, Earl Eugene**
**805 Oak Court R.R.7**
**Greenfield Indiana 46140 (US)**
Inventor: **Gordee, Robert Stouffer**
**5166 East 74th Place**
**Indianapolis Indiana 46250 (US)**
Inventor: **Hull, Robert Normond**
**391, Leisure Lane**
**Greenwood Indiana 46142 (US)**

(74) Representative: **Crowther, Terence Roger et al,**
**Erl Wood Manor**
**Windlesham Surrey GU20 6PH (GB)**

(56) References cited:
**UNLISTED DRUGS, vol. 23, no. 4, April 1971,**
**page 49 Chatham, N.J. USA**
**UNLISTED DRUGS, vol. 21, no. 10, October**
**1969, page 156 Chatham. N.J. USA**

Courier Press, Leamington Spa, England.

## Description

This invention relates to certain novel combinations of macrolide and aminoglycoside antibiotics which have been found to be particularly effective in the treatment of colibacillosis in pigs and are also valuable as growth promoters and in improving feed utilization in that species. They also have value in the control of mycoplasma growth in mammalian tissue cultures.

The macrolide and aminoglycoside antibiotics comprising the compositions are all known. Tylosin, U.S. Patent 3,178,341, Merck Index 9th Ed., No. 9486 is sold commercially as a veterinary antibiotic; spiromycin is disclosed in U.S. Patent 2,943,023, Merck Index 9th Ed., No. 8525; leucomycin is disclosed in Merck Index 9th., No. 5307; magnamycin is disclosed in U.S. Patent 2,960,438 and Merck Index 9th Ed., No. 1812 and oleandomycin is disclosed in U.S. Patent Nos. 2,757,123 and 2,842,481 and Merck Index 9th Ed., No. 6671.

The aminoglycoside antibiotics tobramycin and apramycin are alternatively known as nebramycin factors 6 and 2 respectively. Tobramycin, apramycin and nebramycin factor 5 are members of the nebramycin complex of antibiotics produced by *Streptomyces tennibrarius* and are described in U.S. Patent 3,691,279. Tobramycin is used clinically in human medicine. Neomycin is disclosed in U.S. Patent 2,799,620 while gentamicin is disclosed in U.S. Patent 3,091,572 and Merck Index 9th Ed., No. 4223.

A preparation containing *inter alia* neomycin and oleandomycin for the treatment of skin infections is described under the name "Sofan" in "Unlisted Drugs" Vol. 29, No. 6, p. 102, while a combination of leucomycin and neomycin is known from Derwent Japanese Patent Report, Vol. 5, No. 45, abstract No. 722145, for use in silkworm disease control.

According to the present invention there is provided an antibiotic composition comprising a macrolide antibiotic selected from the group consisting of tylosin, leucomycin, magnamycin, spiromycin and oleandomycin or a physiologically acceptable salt thereof and an aminoglycoside antibiotic selected from the group consisting of tobramycin, apramycin, nebramycin Factor 5, gentamicin and neomycin or a physiologically acceptable salt thereof with the proviso that when the aminoglycoside antibiotic is neomycin the macrolide antibiotic is not leucomycin or oleandomycin.

The invention also provides a method for promoting the growth or improving feed utilization in pigs by administering thereto an antibiotic composition as described above.

The invention also provides a method for controlling the growth of mycoplasma in mammalian tissue culture by applying to said tissue culture an antibiotic composition described above.

The invention also provides a method for treating colibacillosis in pigs which comprises administering to said pigs an antibiotic composition as described above.

The antibiotic compositions of this invention are useful in the control of mycoplasma and in particular mycoplasma that are resistant to the individual antibiotics. The synergism of the compositions was demonstrated in mammalian tissue cultures where mycoplasma are frequent and often unrecognized contaminants. The mycoplasma contamination of tissue culture preparations of various cell lines, for example in the propagation of animal virus, can ruin the culture preparation and in some instances after culture has been maintained for several weeks. The synergistic activity of the compositions of this invention is best demonstrated against resistant mycoplasma in mammalian tissue culture. The term, "resistant mycoplasma", as used herein refers to mycoplasma which are resistant to both the macrolide antibiotics and the aminoglycoside antibiotics comprising the compositions of this invention at minimal inhibitory concentrations higher than those generally observed with the antibiotics against various microorganisms. A number of mycoplasma are resistant to the antibiotics comprising the compositions of this invention. Prevalent among resistant mycoplasma contaminants in mammalian tissue culture preparations are strains of *M, hyorhinis. M. gallisepticum* resistant strains are also frequent contaminants. The synergistic compositions of this invention inhibit the growth of resistant mycoplasma microorganisms at low concentrations when the individual antibiotics are ineffective alone at high concentrations. The following Table 1 lists the minimal inhibitor concentrations (MIC) in $\mu g/ml$ for the individual macrolide and aminoglycoside antibiotics and their combinations. The MIC values were obtained with mammalian tissue cultures contaminated with the designated mycoplasma in experiments carried out as follows.

Suspensions of monkey kidney cells, LLC-MK2, in medium 199 (Morgan et al., *Proc. Soc. Exp. Biol. Med, 73,* 1 1950), were inoculated with cultures of the indicated mycoplasma to achieve an inoculum concentration of $1.5 \times 10^7$ organisms/ml of cell suspension. Control cultures and the mycoplasma positive cultures included in each test were incubated at 37°C for 3—5 days. After incubation the medium in all cultures was changed to 1% horse serum in Medium 199 containing 1.68 g of sodium bicarbonates per liter. The tissue cultures innoculated with mycoplasma (except for mycoplasma positive control cultures) were then treated with the indicated individual antibiotics alone or with the combination of antibiotics shown in Table 1. When combinations of antibiotics were tested equal amounts of the antibiotics were used.

Solutions of the antibiotics were made up in Medium 199 containing 1.68 g of sodium bicarbonate per liter of medium. The solutions were sterile filtered through a millipore filter having a 0.2 $\mu$m porosity and were stored at 4°C prior to use. Two fold dilutions of the antibiotic solutions were made and each antibiotic solution was added to separate cultures to determine the minimal inhibitory concentration. The medium in each culture was changed twice a week with fresh antibiotic solution at the same concentration being added at each medium change. After two weeks, medium changes were made without antibiotic for two more weeks. Samples of the cultures were taken before each medium change in the last two weeks and were cultured to determine the presence of mycoplasma.

TABLE 1

Minimal inhibitor concentrations of antibiotics vs mycoplasma in LLC-MK2 tissue culture

| Antibiotic[1] | Mycoplasma | Minimal inhibitory concentration ($\mu$g/ml) |
|---|---|---|
| Tylosin | M. Hyorhinis HH[2] | >250 |
| Tobramycin | M. Hyorhinis HH[2] | 62.5 |
| Combination | M. Hyorhinis HH[2] | <15.6 |
| Tylosin | M. Hyorhinis | 100 |
| Tobramycin | M. Hyorhinis | 100 |
| Combination | M. Hyorhinis | 3 |
| Tylosin | M. Hyorhinis HEK[3] | >200 |
| Tobramycin | M. Hyorhinis HEK[3] | >500 |
| Combination | M. Hyorhinis HEK[3] | 3.1 |
| Tylosin tartrate | M. Hyorhinis HH | 200 |
| Tobramycin sulfate | M. Hyorhinis HH | 200 |
| Combination | M. Hyorhinis HH | 1.5 |
| Tylosin tartrate | M. gallisepticum 36 F[4] | >800 |
| Tobramycin | M. gallisepticum 36 F[4] | 100 |
| Combination | M. gallisepticum 36 F[4] | 25 |
| Tylosin | gallisepticum 41313[5] | 800 |
| Tobramycin | M. gallisepticum 41313[5] | 200 |
| Combination | M. gallisepticum | 12.5 |
| Tylosin | Swine L3F12F7[6] | 200 |
| Tobramycin | Swine L3F12F7[6] | 50 |
| Combination | Swine L3F12F7[6] | <1.5 |
| Tylosin | M. Hyorhinis HH | 200 |
| Apramycin | M. Hyorhinis HH | 600 |
| Combination | M. Hyorhinis HH | 12 |
| Tylosin | M. Hyorhinis | 400 |
| Gentamicin | M. Hyorhinis | 100 |
| Combination | M. Hyorhinis | 0.78 |
| Tylosin | M. Hyorhinis HH | >800 |
| Nebramycin Factor 5 | M. Hyorhinis HH | 200 |
| Combination | M. Hyorhinis HH | 25 |
| Tylosin | M. Hyorhinis | 200 |
| Neomycin | M. Hyorhinis | 100 |
| Combination | M. Hyorhinis | 6.3 |
| Spiromycin | M. Hyorhinis HH | >800 |
| Tobramycin | M. Hyorhinis HH | 200 |
| Combination | M. Hyorhinis HH | 1.5 |
| Magnamycin | M. Hyorhinis HH | >800 |
| Tobramycin | M. Hyorhinis HH | 200 |
| Combination | M. Hyorhinis HH | 1.5 |

3

**O 032 304**

TABLE 1 (contd.)

| Antibiotic[1] | Mycoplasma | Minimal inhibitory concentration ($\mu$g/ml) |
|---|---|---|
| Leucomycin | M. Hyorhinis HH | >400 |
| Tobramycin | M. Hyorhinis HH | 200 |
| Combination | M. Hyorhinis HH | 0.78 |
| Oleandomycin | M. Hyorhinis HH | 400 |
| Tobramycin | M. Hyorhinis HH | 200 |
| Combination | M. Hyorhinis HH | 100 |

[1]Combinations of the indicated antibiotics contained equal amounts of each antibiotic.
[2]M. Hyorhinis H.H. is an isolate from human heart tissue.
[3]M. Hyorhinis HEK is an isolate from human embryonic kidney tissue.
[4]M. Gallisepticum 36F is a strain of a chicken mycoplasma.
[5]M. Gallisepticum 41313 is a strain of a chicken mycoplasma.
[6]Swine L3F12F7 was a mycoplasma isolated from swine and from its characteristics appears to be *Mycoplasma arginini*.

As is shown in Table 1 the mycoplasma employed in the test were controlled only at high concentrations by the individual antibiotics. However when one of the macrolide-aminoglycoside compositions of this invention was used the mycoplasma were controlled at greatly reduced concentrations. In each combination the individual antibiotics were present in equal parts.

The compositions of this invention are non-toxic to mammalian tissue and can be used to maintain cell lines free of contamination by mycoplasma. The compositions can also be used in conjunction with other antibiotics which are commonly used in tissue cultures to prevent bacterial contamination but which are ineffective in controlling mycoplasma. For example the synergistic compositions can be used with a penicillin such as penicillin G or with a streptomycin, two antibiotics commonly used in tissue cultures.

A preferred composition of this invention is tylosin-tobramycin. This composition has consistently exhibited low minimal inhibitory concentrations against mycoplasma which tylosin or tobramycin alone control only at high minimal inhibitory concentrations. Table 2 which follows contains the results obtained with numerous combinations of tylosin and tobramycin which inhibited the growth of *M. hyorhinis* in tissue culture preparations using monkey kidney cells LLC-MK2. The tests were conducted in the following manner.

Tissue cultures of LLC-MK2 cells were inoculated with *M. hyorhinis* culture five days prior to the addition of tylosin-tobramycin combinations containing varying proportions of the antibiotics and at various concentrations. Medium changes were made at three to five-day intervals with antibiotics addition at each change. Samples of the culture media were taken at each media change and assayed for mycoplasma. The data in Table 2 illustrate various proportions of the antibiotics in combinations which inhibited the growth of *M. hyorhinis*.

TABLE 2

Inhibition of *M. hyorhinis* in LLC-MK2 tissue culture by tylosin-tobramycin compositions

| Tobramycin ($\mu$g/ml) | plus | Tylosin ($\mu$g/ml) |
|---|---|---|
| 12 | | 1 |
| 6 | | 1 |
| 3 | | 1 |
| 2 | | 2 |
| 1 | | 6 |
| 0.5 | | 12 |

All of the combinations of tylosin and tobramycin shown in Table 2 inhibited the growth of mycoplasma for from five to eight samplings covering a period of three and a half weeks. When used alone tylosin and tobramycin exhibit much higher MIC values as shown in Table 1.

The data in Table 2 demonstrate the low concentrations at which the antibiotic compositions of this invention are synergistically effective. Compositions containing from one part of tobramycin or greater to 3 parts of tylosin or greater by weight are preferred synergistic compositions.

Another preferred composition of this invention comprises tylosin in combination with apramycin.

4

As is shown in Table 1, the solution containing 12 μg/ml of tylosin and 12 μg/ml of apramycin inhibited mycoplasma whereas the MIC for apramycin alone was 600 μg/ml and that for tylosin alone was 200 μg/ml.

A further preferred composition comprises tylosin in combination with gentamicin.

In contrast to the synergism demonstrated by the macrolide antibiotic-aminoglycoside antibiotic compositions of this invention a number of macrolide-macrolide antibiotic combinations showed little if any enhanced activity in the control of mycoplasma in tissue cultures.

The macrolide and aminoglycoside antibiotics forming the synergistic compositions can be in a suitable acid addition salt form or as the free bases. These antibiotics form salts with mineral acids and carboxylic acids. Examples of such salts are tylosin hydrochloride, tylosin tartrate, tylosine phosphate, spiromycin adipate, spiromycin hydrochloride, oleandomycin hydrochloride, leucomycin hydrobromide, magnamycin tartrate, tobramycin sulfate, apramycin hydrochloride, neomycin sulfate, neomysin hydrochloride, gentamicin hydrochloride, and gentamicin sulfate.

The antibiotic formulations of this invention can be prepared by mixing the dry antibiotic powders of the free base or a salt form thereof or, alternatively solutions of the antibiotics of the appropriate concentration can be mixed to obtain a solution of the two antibiotics of the desired concentration. The individual antibiotics are appreciably soluble in aqueous media and solutions of the formulations in aqueous tissue culture medium are desirably used in controlling mycoplasma contamination in mammalian tissue cultures.

The synergistic antibiotic compositions of this invention can be used to inhibit the growth of mycoplasma in a wide variety of mammalian tissue and thus prevent the contamination and loss thereof. For example, a composition of the invention can be employed in mammalian tissue cultures used in the preparation of plasminogen activator according to the method described by Hull et al., U.S. Patent 3,904,480.

The antibiotic compositions of this invention generally comprise the macrolide antibiotic in the ratio by weight to the aminoglycoside antibiotic of from 15:1 to 1:15. A preferred ratio by weight of the macrolide to the aminoglycoside is 3:1 to 1:3.

A preferred antibiotic composition of this invention, tylosin in combination with apramycin, is also useful in enhancing the growth in post weaned pigs. The tylosin-apramycin combination demonstrates higher feed utilization and higher weight gains in post weaned pigs than does either antibiotic when administered alone. The efficacy of the tylosin-apramycin combination in enhancing feed utilization and weight gain in post weaned pigs was determined in animal trials carried out as follows.

The trials were carried out in pens with 6 pigs per pen for 28 days. The pigs were of mixed sex having an average initial body weight of 12.89 kg and an average age of six weeks. The animals received water before and after feeding. The animals were fed twice daily with feed having the following composition.

| Ingredient | Percent |
| --- | --- |
| Wheat | 21 |
| Barley | 23.5 |
| Corn | 20 |
| Fish meal | 2.5 |
| Yeast | 1 |
| Wheat bran | 10 |
| Alfalfa | 2.5 |
| Soybean meal | 14 |
| Oat hulls | 1.8 |
| Calcium phosphate | 1 |
| Calcium carbonate | 1.1 |
| Sodium chloride | 0.1 |
| Premix SV12 | 1 |
| Premix SO4 | 0.5 |

The tylosin-apramycin combination was compared in the trials with tylosin alone and with apramycin alone. The antibiotics and the combination thereof were administered in the animal feed at a level of 100 ppm, and, in the case of the combination; at 100 ppm for each antibiotic.

The combined results of two like trials are shown below in Table 3.

TABLE 3

Comparative weight gains and feed utilization in pigs treated with apramycin-tylosin, tylosin and apramycin

| Treatment | Negative control | Tylosin | Apramycin | Tylosin-apramycin |
|---|---|---|---|---|
| Conc. in Feed (ppm) | — | 100 | 100 | 100—100 |
| Number of animals n | 70 | 71 | 72 | 72 |
| Number of replic. n | 12 | 12 | 12 | 12 |
| Initial weight $\bar{x}$ | 13,11 | 13,03 | 12,55 | 12,85 |
| (kg) per animal ±s | 0,81 | 1,04 | 0,58 | 0,71 |
| v | 6 | 8 | 5 | 6 |
| (Rel) | (100) | (99) | (96) | (98) |
| Final weight $\bar{x}$ | 25,00 | 25,58 | 25,62 | 26,71 |
| (kg) per animal ±s | 1,48 | 1,65 | 1,14 | 1,49 |
| v | 6 | 6 | 4 | 6 |
| (Rel) | (100) | (102) | (102) | (107) |
| Daily gain $\bar{x}$ | 425 | 449 | 467 | 495 |
| (g) per animal ±s | 40 | 62 | 38 | 44 |
| v | 9 | 14 | 8 | 9 |
| (Rel) | (100) | (106) | (110) | (116) |
| Daily feed intake $\bar{x}$ | 0,92 | 0,92 | 0,92 | 0,94 |
| (kg) per animal ±s | 0,06 | 0,06 | 0,04 | 0,05 |
| v | 7 | 6 | 4 | 5 |
| (Rel) | (100) | (100) | (100) | (102) |
| Feed efficiency $\bar{x}$ | 2,14 | 2,07 | 1,96 | 1,90 |
| (kg feed/kg gain) ±s | 0,10 | 0,17 | 0,09 | 0,09 |
| v | 4 | 8 | 4 | 5 |
| (Rel) | (100) | (97) | (92) | (89) |

In Table 3 the statistical symbols have the following meanings: $\bar{x}$=means; ±s=standard deviation; v=coefficient of variances; and (Rel)=comparison treatments to negative controls in percentage.

As shown in Table 3 the pigs treated with tylosin-apramycin combination showed higher weight gains and better feed efficiency on the 28 day trial than did the pigs treated with tylosin alone or with apramycin alone.

In a second test the efficacy of the tylosin-apramycin combination was demonstrated in controlled studies in weanling pigs. The results show that pigs treated with the antibiotic combination had a significantly greater reduction in the incidence of *E. coli* diarrhoea than did the pigs treated with apramycin alone or with tylosin alone. Further the pigs treated with the antibiotic compositions showed higher average daily weight gains and greater improved feed efficiency than did pigs treated with either antibiotic alone.

The following Table 4 shows the results obtained in a 21 day study with weanling pigs. Tylosin and apramycin were each administered in the feed at a level of 110 ppm. The antibiotic combination was likewise administered in the feed containing both antibiotics at the 110 ppm level. The results are shown as a percentage increase over a non-treated control group as calculated from the statistical evaluation of the data obtained in the study.

TABLE 4

| | Percent increase over controls | | |
|---|---|---|---|
| Treatment | Average daily weight gain | Improved feed efficiency | Reduction of diarrhoea |
| Apramycin | 19.0 | 10.3 | 42.8 |
| Tylosin | 29.7 | 11.4 | 26.4 |
| Tylosin/Apramycin | 40.6 | 16.1 | 48.7 |

As shown in Table 4 tylosin when used alone was effective in improving feed efficiency, weight gain and in reducing the incidence of diarrhoea. Apramycin when used alone likewise showed its known effectiveness in each category. Surprisingly, the use of both antibiotics in combination showed greater improvement in each category than did the use of apramycin alone. As noted above, tylosin was not previously known to be effective in the treatment of colibacillosis in post weaned pigs and, consequently the greater effectiveness of the tylosin-apramycin combination over that displayed by apramycin was unexpected.

The method of this invention comprises administering to weanling pigs a therapeutically effective amount of tylosin and apramycin. The total amount of both antibiotics as well as the proportionate amounts of each which may be administered can vary. The effective concentration of the antibiotics in the animal's feed can depend on such factors as the severity of the disease, and the age and weight of the animals. The concentration of both antibiotics together in the feed can generally be between 50 ppm and 1000 ppm with the preferred ratio by weight of tylosin to apramycin of from 5:1 to 1:5. A generally useful concentration of both antibiotics together in feed is between 100 ppm and 300 ppm. A preferred concentration is about 220 ppm. A most preferred ratio by weight of the antibiotics is 1:1.

Tylosin and apramycin are basic substances which form salts with mineral acids, carboxylic acids and sulfonic acids such as the hydrochloride, sulfate, phosphate, tartrate, citrate, laurylsulfate, hexanoate, benzene sulfonate, and like salts. The salt form of the antibiotics as well as the free base form can be employed in the method of this invention. Preferred salts of tylosin are the tartrate and the phosphate salts. Preferred salts of apramycin are the sulfate and hydrochloride salts.

The tylosin and apramycin can be formulated together in a premix or in separate premixes for mixing with the final feed. Alternatively, the two antibiotics can be mixed directly into the final feed.

The antibiotics in the base form are appreciably soluble in water as are the salts of the antibiotics. The antibiotics and preferably the salt forms thereof can be added to the animal's drinking water to form solutions at therapeutically effective concentrations. As with the concentration in the animal's feed the concentration of the antibiotics in drinking water may be varied. Concentrations of both antibiotics of between 10 $\mu$g/ml to 500 $\mu$g/ml, with a ratio of the antibiotics by weight of 5:1 to 1:5, are generally sufficient to provide control and treatment in the animals. A preferred concentration of the antibiotics in the drinking water is between 50 $\mu$g/ml to 300 $\mu$g/ml.

While the antibiotic compositions of tylosin and apramycin are most practically administered in the animal's feed or drinking water the compositions are also effective when injected into the pigs. For administration by injection the two antibiotics can be formulated with a suitable pharmaceutically acceptable diluent such as water, physiological saline or dextrose. The administration of the tylosin-apramycin compositions by injection can be used to treat weanling pigs which are too debilitated by the disease to eat or to consume a sufficient amount of the medicated feed. In such instances the antibiotic compositions can be administered to the pigs by daily injection. Alternatively the antibiotics can be formulated in suitable oral dosage forms, for example in tablets, capsules, or suspensions, and administered to pigs which are incapable of eating a sufficient quantity of the medicated feed.

The efficacy of the tylosin-apramycin combination in the treatment and control of colibacillosis is shown by the results obtained in a 21 day trial with weanling pigs. The pigs were divided by weight into treatment and control groups of eight pigs. Each treatment was randomly assigned to three groups. The animals were fed a normal ration *ad libitum* with the treatment groups receiving feed containing 110 ppm of each antibiotic. Weight gains, feed efficiency and the incidence of diarrhoea were used to measure the response to treatment. The results obtained after 21 days are shown in Table 5.

TABLE 5

Tylosin-apramycin 21 day treatment of weanling pigs

| Treatment[1] | Average daily gain (kg) | Average daily feed (kg) | Feed efficiency | Diarrhoea[2] index |
|---|---|---|---|---|
| Control | 0.23 | 0.42 | 1.81 | 2.84 |
| Apramycin | 0.29 | 0.47 | 1.64 | 1.64 |
| Tylosin | 0.31 | 0.51 | 1.62 | 2.10 |
| Tylosin/Apramycin | 0.34 | 0.51 | 1.53 | 1.46 |

[1]/Tylosin phosphate and apramycin sulfate at 110 ppm in feed. Tylosin-apramycin combination at 110 ppm of each antibiotic in feed.

[2]/The diarrhoea index was based on the following system with scoring twice daily for the first 13 days and once on the 14th day.

1. Formed stools only.
2. Formed stools, <50% unformed.
3. Unformed stools, >50% unformed.
4. Unformed stools only.
5. Liquid stools only.

7

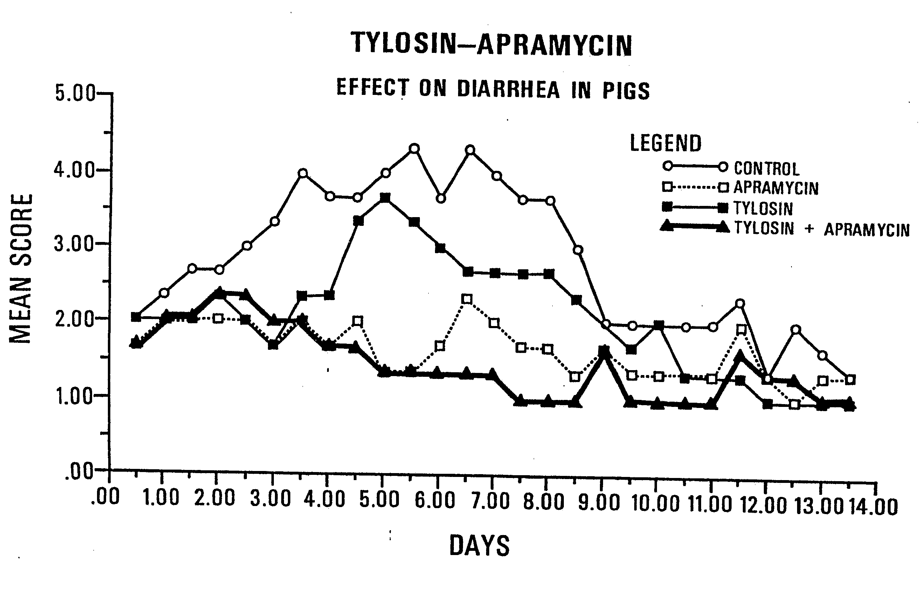

The accompanying drawing graphically shows the plot of the mean diarrhoea scores vs time in days obtained in the above study. Plotted are the mean scores obtained with the control group and the treated groups. As shown by the plot of the mean scores for the group treated with the tylosin-apramycin combination the incidence of diarrhoea was significantly lower than the incidence for the group treated with apramycin alone. The graph also shows that tylosin alone was of some effectiveness in reducing the incidence of diarrhoea.

In carrying out the method of this invention the weanlings are treated with the antibiotic combination from the time of weaning to 3—4 weeks post-weaning. In the case of severe disease incidence the treatment can be maintained for a longer time if necessary.

The data presented in Table 4 above show the difference in the incidence of diarrhoea between the treated groups and the control group expressed as the percentage of the control incidence.

The tylosin-apramycin compositions of this invention demonstrate synergistic activity against strains of *Pasturella hemolytica.* This organism can cause pneumonia in cattle which in cases of severe infection can lead to death. The synergism of the tylosin-apramycin combination was demonstrated *in vitro* against *P. hemolytica* strain 41D. Tylosin alone had a minimal inhibitory concentration of 50 $\mu$g/ml and that for apramycin alone was 25 $\mu$g/ml. The synergistic activity of the combination of the two antibiotics was determined by the "checkerboard" titration method. In this method two antibiotics are tested in serial dilutions and in all combinations of these dilutions together to find the concentrations of each antibiotic, both alone and in combination, that inhibit growth of the test microorganism. The nature of the interaction between the two antibiotics may be determined algebraically or geometrically. In the algebraic method, the concentration of each antibiotic in the combination that inhibits growth is expressed as a fraction of the concentration that produces the same effect when the antibiotic is used alone. When the sum of these fractions is one, the combination is additive, when the sum is less than one, the combination is synergistic; and when the sum is greater than one, the combination is antagonistic.

Below are shown the calculations using data obtained in the in vitro test of tylosin, apramycin and the combination of the two antibiotics against *P.* hemolytica.

Tylosin alone: MIC=50 $\mu$g/ml
apramycin: MIC=25 $\mu$g/ml
Tylosin+Apramycin
25/50+0.8/25=<1
12.5/50+6.25/25=<1
6.25/50+12.5/25=<1

The following antibiotic compositions are preferred compositions

Tylosin-Tobramycin
Tylosin-Apramycin
Tylosin-Gentamicin
Leucomycin-Tobramycin
Spiromycin-Tobramycin
Magnamycin-Tobramycin

Especially preferred compositions are tylosin-tobramycin, tylosin-apramycin, and tylosin-gentamicin.

The antibiotic compositions of this invention are, as described hereinabove, valuable in veterinary medicine in the treatment of various animal diseases. The compositions are also valuable in controlling mycoplasma contamination in the propagation of virus by the tissue culture method.

The following examples further illustrate the present invention.

Example 1

Cell suspensions of LLC-MK2 (Monkey kidney cell line) in Medium 199 containing 1% house serum of 4 ml volumes in tissue culture flasks were inoculated with a 3 day old culture of *Mycoplasma hyorhinis* ($5 \times 10^8$ organism per ml in Eaton's broth). Approximately 2 to 3% of the mycoplasma culture per volume of the cell suspension was used. After inoculation the cell suspensions were incubated at a temperature of 37°C for from three to five days. Control cultures were also made up in the same manner. After incubation the medium in the control cultures and the mycoplasma positive cultures was replaced with fresh Medium 199 containing 1.68 g of sodium bicarbonate and 1% horse serum. The cultures were then treated with solutions of tylosin, tobramycin, and with the combination of tylosin and tobramycin in Medium 199 containing 1.68 g of sodium bicarbonate per liter. Solutions of each individual antibiotic and of the combination at a concentration of 1000 $\mu$g/ml were prepared. The solution of the combination had a concentration of each antibiotic of 1000 $\mu$g. By two-fold serial dilution solutions at various concentrations down to 0.39 $\mu$g/ml were prepared. Each of the antibiotic solutions was added to a separate flask containing the tissue culture.

8

## 0 032 304

The tissue culture medium was replaced in each flask twice a week and at each medium change for two weeks a fresh solution of the antibiotic at the same concentration was added. After two weeks fresh medium replacements were made for two more weeks without the addition of antibiotic. Samples of each culture were taken before each medium replacement and were cultured to determine the presence of mycoplasma. The minimal inhibitory concentration of the individual antibiotics and of the combination, tylosin and tobramycin, was the lowest concentration of the antibiotic in a given tissue culture which failed to culture for mycoplasma on sampling. The minimum inhibitory concentration for tylosin alone was 100 $\mu$g/ml and for tobramycin alone was 100 $\mu$g/ml while for the combination the MIC was 3 $\mu$g/ml.

Example 2

When tylosin, apramycin, and the tylosin-apramycin combinations were evaluated in tissue culture contaminated with *M. hyorhinis* H.H. according to the method and procedures employed as described by Example 1 tylosin alone had an MIC of 200 $\mu$g/ml, apramycin alone had an MIC of 600 $\mu$g/ml while the tylosin-apramycin combination had an MIC of 12 $\mu$g/ml.

Example 3

By employing *M. hyorhinis* in the procedure of Example 1 and substituting gentamicin for tobramycin and tylosin-gentamycin for the tylosin-tobramycin combination, tylosin alone had an MIC of 400 $\mu$g/ml. The combination of tylosin-gentamicin had an MIC of 0.78 $\mu$g/ml.

Example 4

The combination, leucomycin-tobramycin, was substituted for the tylosin-tobramycin combination used in Example 1. Leucomycin alone had an MIC of 400 $\mu$g/ml, tobramycin an MIC of 200 mcg/ml while the combination had an MIC of 0.78 $\mu$g/ml.

Example 5

Ninety-six weanling pigs averaging 6.0 kg in weight were randomly allocated by weight into 12 pens of 8 pigs per pen. Four treatments were used in the study as follows: 1) Non-medicated controls; 2) apramycin fed at 110 ppm; 3) tylosin phosphate fed at 110 ppm; and 4) apramycin sulfate and tylosin phosphate both fed at 110 ppm. Each treatment was replicated through three pens.

The pigs were fed for 21 days *ad libitum* a ration of 20% protein corn-soy meal with some rolled oats included for roughage. Vitamins and minerals were added as needed to balance the ration. The only difference in the rations fed to all pigs was the inclusion of the appropriate antibiotic or antibiotic combination in each of the medicated rations. All feed was weighed as fed, with the feed remaining at the end of the weigh period, weighed and discarded. The pigs were weighed at the initiation of the study and weekly thereafter.

Diarrhoea scores were maintained on a pen basis. The scoring was done by the same person on a twice daily schedule for the first 13 days and once only on the 14th day. The scoring index was the same as that shown in footnote 2 of Table 5.

An extra pen of pigs was maintained for bacteriological surveys. These pigs were sacrificed as diarrhoea was first observed as occurring following allocation of animals to individual pens and prior to the feeding of any medicated rations.

The number of beta hemolytic *E. coli* per ml of duodenum or ileum contents was determined for the sacrificed animals. The results demonstrated the presence of *E. coli* at levels of $10^7$ and $10^8$ in the duodenum of the two animals assayed. Negative results were obtained on culturing for salmonella.

The results of the study expressed in weight gain, reduced incidence of diarrhoea and improved feed efficiency are shown in Tables 4 and 5 herein.

## Claims

1. An antibiotic composition comprising a macrolide antibiotic selected from the group consisting of tylosin, leucomycin, magnamycin, spiromycin and oleandomycin or a physiologically acceptable salt thereof and an aminoglycoside antibiotic selected from the group consisting of tobramycin, apramycin, nebramycin Factor 5, gentamicin and neomycin or a physiologically acceptable salt thereof with the proviso that when the aminoglycoside antibiotic is neomycin the macrolide is not leucomycin or oleandomycin.

2. An antibiotic composition as claimed in claim 1, wherein the macrolide antibiotic is tylosin or a physiologically acceptable salt thereof.

3. An antibiotic composition as claimed in claim 1 or 2, wherein the aminoglycoside antibiotic is tobramycin or a physiologically acceptable salt thereof.

4. An antibiotic composition as claimed in claim 1 or 2, wherein the aminoglycoside antibiotic is apramycin or a physiologically acceptable salt thereof.

5. An antibiotic composition as claimed in claim 1 comprising tylosin or a physiologically acceptable salt thereof and tobramycin or a physiologically acceptable salt thereof.

9

6. An antibiotic composition as claimed in any one of claims 1 to 5 for use as a growth promoter or to improve feed utilization in pigs.

7. An antibiotic composition as claimed in any one of claims 1 to 5 for use in controlling the growth of mycoplasma in mammalian tissue culture.

8. An antibiotic composition as claimed in any one of claims 1 to 5 for use in treating colibacillosis in pigs.

9. An antibiotic composition as claimed in claim 8, wherein the macrolide is tylosin or a physiologically acceptable salt thereof and the aminoglycoside is apramycin or a physiologically acceptable salt thereof.

10. An antibiotic composition as claimed in any one of claims 1 to 9 which comprises tylosin phosphate and apramycin sulfate.

11. A method for promoting the growth or improving feed utilization in pigs by administering thereto an antibiotic composition as claimed in any of claims 1 to 5.

12. A method for controlling the growth of mycoplasma in mammalian tissue culture by applying to said tissue culture an antibiotic composition as claimed in any one of claims 1 to 5.

## Patentansprüche

1. Antibiotische Zusammensetzung, gekennzeichnet durch einen Gehalt an einem Makrolidantibiotikum aus der Gruppe Tylosin, Leucomycin, Magnamycin, Spiromycin und Oleandomycin oder einem physiologisch annehmbaren Salz hiervon und einem Aminoglykosidantibiotikum aus der Gruppe Tobramycin, Apramycin, Nebramycin Faktor 5, Gentamycin und Neomycin oder einem physiologisch annehmbaren Salz hiervon, mit der Maßgabe, daß das Makrolidantibiotikum nicht Leucomycin oder Oleandomycin ist, falls das Aminoglykosidantibiotikum Neomycin ist.

2. Antibiotische Zusammensetzung nach Anspruch 1, dadurch gekennzeichnet, daß das Makrolidantibiotikum Tylosin oder ein physiologisch annehmbares Salz hiervon ist.

3. Antibiotische Zusammensetzung nach Anspruch 1 oder 2, dadurch gekennzeichnet, daß das Aminoglykosidantibiotikum Tobramycin oder ein physiologisch annehmbares Salz hiervon ist.

4. Antibiotische Zusammensetzung nach Anspruch 1 oder 2, dadurch gekennzeichnet, daß das Aminoglykosidantibiotikum Apramycin oder ein physiologisch annehmbares Salz hiervon ist.

5. Antibiotische Zusammensetzung nach Anspruch 1, dadurch gekennzeichnet, daß sie Tylosin oder ein physiologisch annehmbares Salz hiervon und Tobramycin oder ein physiologisch annehmbares Salz hiervon enthält.

6. Verwendung einer antibiotischen Zusammensetzung nach einem der Ansprüche 1 bis 5 als Mittel zur Wachstumsförderung oder zur Verbesserung der Futterausnutzung bei Schweinen.

7. Verwendung einer antibiotischen Zusammensetzung nach einem der Ansprüche 1 bis 5 zur Bekämpfung des Wachstums von Mycoplasma in Gewebekulturen von Säugetieren.

8. Verwendung einer antibiotischen Zusammensetzung nach einem der Ansprüche 1 bis 5 zur Behandlung von Colibacillose bei Schweinen.

9. Verwendung nach Anspruch 8, dadurch gekennzeichnet, daß die antibiotische Zusammensetzung als Makrolidantibiotikum Tylosin oder ein physiologische annehmbares Salz hiervon und als Aminoglykosidantibiotikum Apramycin oder ein physiologisch annehmbares Salz hiervon enthält.

10. Antibiotische Zusammensetzung oder ihre Verwendung nach einem der Ansprüche 1 bis 9, dadurch gekennzeichnet, daß das Makrolidantibiotikum Tylosinphosphat und das Aminglykosidantibiotikum Apramycinsulfat ist.

11. Verfahren zur Erhöhung des Wachstums oder Verbesserung der Futterverwertung bei Schweinen, dadurch gekennzeichnet, daß man Schweinen eine antibiotische Zusammensetzung nach einem der Ansprüche 1 bis 5 verabreicht.

12. Verfahren zur Bekämpfung des Wachstums von Mycoplasmen bei Gewebekulturen von Säugetieren, dadurch gekennzeichnet, daß man eine solche Gewebekultur mit einer antibiotischen Zusammensetzung gemäß einem der Ansprüche 1 bis 5 behandelt.

## Revendications

1. Composition antibiotique comprenant un antibiotique de macrolide choisi parmi le groupe comprenant la tylosine, la leucomycine, la magnamycine, la spiromycine et l'oléandomycine, ou un de ses sels physiologiquement acceptables, ainsi qu'un antibiotique d'aminoglycoside choisi parmi le groupe comprenant la tobramycine, l'apramycine, le facteur 5 de la nébramycine, la gentamycine et la néomycine, ou un de ses sels physiologiquement acceptables, avec cette restriction que, si l'antibiotique d'aminoglycoside est la néomycine, le macrolide ne soit ni la leucomycine, ni l'oléandomycine.

2. Composition antibiotique suivant la revendication 1, caractérisée en ce que l'antibiotique de macrolide est la tylosine ou un de ses sels physiologiquement acceptables.

3. Composition antibiotique suivant la revendication 1 ou 2, caractérisée en ce que l'antibiotique d'aminoglycoside est la tobramycine ou un de ses sels physiologiquement acceptables.

4. Composition antibiotique suivant la revendication 1 ou 2, caractérisée en ce que l'antibiotique d'aminoglycoside est l'apramycine ou un de ses sels physiologiquement acceptables.

5. Composition antibiotique suivant la revendication 1, caractérisée en ce qu'elle comprend la tylosine ou un de ses sels physiologiquement acceptables et la tobramycine ou un de ses sels physiologiquement acceptables.

6. Composition antibiotique suivant l'une quelconque des revendications 1 à 5, en vue de l'utiliser comme accélérateur de croissance ou pour améliorer l'utilisation de la nourriture chez les porcs.

7. Composition antibiotique suivant l'une quelconque des revendications 1 à 5, en vue de l'utiliser pour combattre la croissance de mycoplasme dans la culture des tissus des mammifères.

8. Composition antibiotique suivant l'une quelconque des revendications 1 à 5 en vue de l'utiliser pour le traitement de la colibacillose chez les porcs.

9. Composition antibiotique suivant la revendication 8, caractérisée en ce que la macrolide est la tylosine ou un de ses sels physiologiquement acceptables, tandis que l'aminoglycoside est l'apramycine ou un de ses sels physiologiquement acceptables.

10. Composition antibiotique suivant l'une quelconque des revendications 1 à 9, caractérisée en ce qu'elle comprend le phosphate de tylosine et le sulfate d'apramycine.

11. Procédé pour stimuler la croissance ou améliorer l'utilisation de la nourriture chez les porcs par administration d'une composition antibiotique selon l'une quelconque des revendications 1 à 5.

12. Procédé de contrôle de la croissance de mycoplasme dans la culture de tissus de mammifères en appliquant à cette culture de tissus une composition antibiotique selon l'une quelconque des revendications 1 à 5.

# TYLOSIN—APRAMYCIN
## EFFECT ON DIARRHEA IN PIGS

MEAN SCORE

DAYS

LEGEND
○——○ CONTROL
□·······□ APRAMYCIN
■——■ TYLOSIN
▲——▲ TYLOSIN + APRAMYCIN

0 032 304